# EUROPEAN PATENT APPLICATION

(11) **EP 1 844 750 A1**
(43) Date of publication of application: **17.10.2007**
(21) Application number: 06425261.2
(22) Date of filing: 12.04.2006
(51) Int. Cl.: A61H 23/02

(54) **Device for the treatment of cellulite and adipose tissue**

(71) Applicant: Lain Electronic S.r.L., 20011 Corbetta MI (IT)
(72) Inventor: Marzorati, Armando, 20010 Santo Stefano Ticino (Milano) (IT)
(74) Representative: Lunati, Vittoriano

(57) **Abstract**

A method is provided for the aesthetic treatment of cellulite and adipose tissues present in parts of human or animal bodies, consisting of the application of ultrasounds at frequencies comprised of between 25 kHz and 50 kHz and suitable for generating cavitation within the aqueous liquids in said body parts; furthermore, a device is provided for carrying out said method consisting of an alternating electrical current generating device (10), at least one transducer (30), electrically connected to the generating device (10) and suitable for transforming the alternating electrical current into mechanical vibrations thus generating ultrasounds, and means for controlling (12) the generator device.

## Description

This invention relates to a device and a method for the aesthetic treatment of cellulite and adipose tissues, of the type specified in the preamble of the first claim.

As is well known, the term cellulite is used to identify an altered condition of the subcutaneous tissue, characterised by adipose cell hypertrophy, water retention and fluid stasis in the intercellular spaces, and excessive localised adiposity, primarily in the areas of the thighs, buttocks, abdomen and arms.

Cellulite is primarily found in female bodies, and, while for numerous centuries has been considered one of the aesthetic criteria of feminine beauty, it is nowadays considered a blemish.

Cellulite and localised adipose tissues have no influence on a person's state of health or physical wellbeing, however, since these are nowadays considered a blemish, various methods and devices have been devised for the treatment and reduction of cellulite.

In particular, reduction and elimination methods using ultrasounds are known and used in the art.

It is well known that ultrasounds are acoustic vibrations with frequencies higher than the sound frequencies audible to the human ear, i.e. frequencies greater than 15 - 20 kHz.

They are produced artificially using special devices known in the art, equipped with electrical alternating current generators and piezoelectric elements which transform the alternating electrical current vibrations into mechanical vibrations of the appropriate frequency.

Such devices producing said ultrasounds are placed in contact with the areas affected by the presence of cellulite or localised adipose tissues.

The ultrasounds then propagate in the form of compression and decompression waves.

The interaction of ultrasounds with biological tissues can result in mechanical effects, thermal effects, and effects associated with cavitation.

The mechanical effects are due to the particles of the tissues, moving as the ultrasonic wave pass through them.

The thermal effects also depend on the propagation of the ultrasonic waves within the tissues which loose some of their propagation energy, yielding it to the tissues themselves in the form of thermal energy.

The effects associated with cavitation are due to the phenomenon of the same name. As is well known, the phenomenon of cavitation occurs when, in certain areas of a given liquid, such a depression is produced that the localised pressure assumes values which are lower than the vapour pressure. As a consequence, numerous vapour bubbles are formed within the liquid itself. These bubbles are unstable and implode violently.

In the present case, cavitation is due to the rapid movements occurring in close proximity to the piezoelectric element. The aqueous liquids present within tissues, in close proximity to the piezoelectric element, generate small and unstable vapour bubbles.

All the above described effects result in the destruction or reduction of the adipose tissue and cellulite.

Among the various possible treatments for cellulite and adipose tissues, treatment using ultrasounds is frequently preferred; indeed this method is neither invasive nor exacting for the patient.

In particular, ultrasounds with a frequency of around 3 MHz, which is considered optimal and produce the thermal and mechanical effects described, are typically used for the aesthetic treatment of cellulite and adipose tissues.

The known art mentioned above has certain important drawbacks.

Indeed, the results of such applications are barely aesthetically appreciable, and hence not really important.

However, the technical problem of how to create an effective device and method for the aesthetic treatment of cellulite and adipose tissues, which is neither invasive nor exacting for the patient, remains as yet unresolved.

In this situation, the technical aim at the heart of the present invention is to conceive a device and method for the aesthetic treatment of cellulite and adipose tissues, capable of essentially obviating the above-mentioned drawbacks.

Within said technical aim, an important task of the invention is to devise a device and a method for the aesthetic treatment of cellulite and adipose tissues that is both effective and non-invasive.

The technical aim and the specific tasks are achieved by a device and method for the aesthetic treatment of cellulite and adipose tissues as claimed in the appended Claim 1.

Preferred embodiments are described in the sub-claims.

Further features and advantages of the invention are better explained below in the detailed description of a preferred embodiment of the invention, with reference to the attached drawings, wherein:

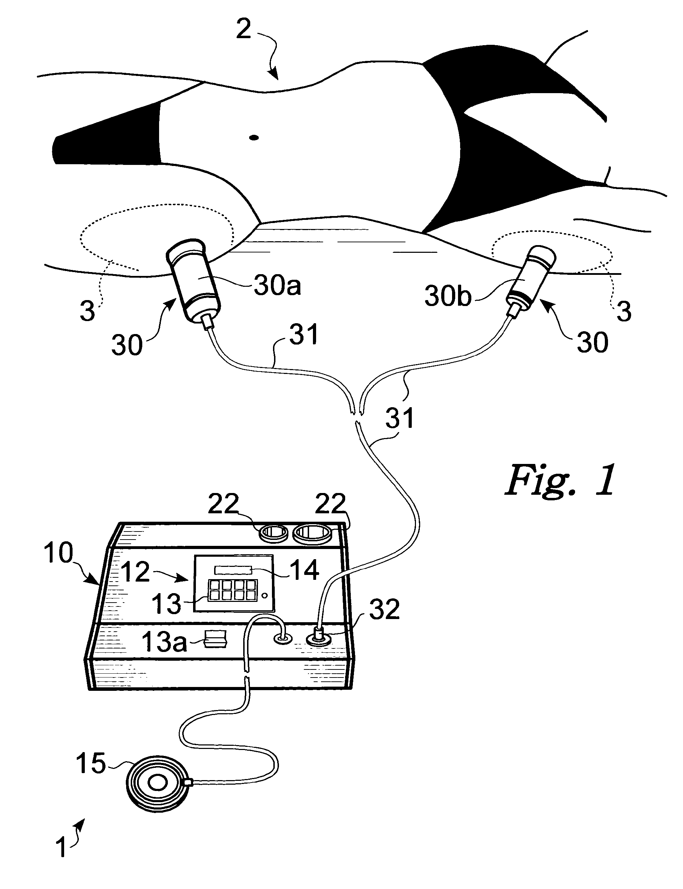

**Fig. 1** shows the device according to the invention in use;
**Fig. 2** is a three-dimensional representation of the device according to the invention;
**Fig. 3a** shows a cross-section, at mid-length, of a part of the device according to the invention;
**Fig. 3b** shows a cross-section, at mid-length, of a variant of a part of the device according to the invention, shown in Fig. 3a; and
**Fig. 4** outlines the interior of a second part of the device according to the invention.

The invention comprises a new method for the aesthetic treatment of cellulite and adipose tissues.

The new method comprises the application of ultrasounds, at a frequency of between 25 kHz and 50 kHz, to adipose tissues and the like. Said ultrasounds are capable of generating cavitation within the aqueous liquids present within adipose tissues and the like.

More particularly, said ultrasounds have frequency values comprised of between 36 kHz and 42 kHz.

Indeed, according to the applicant's research and testing, it has been discovered that, of the three fundamental effects typically caused by the ultrasound frequencies used for the present application: *i.e*. thermal, mechanical and cavitation effects, the cavitation effects give by far and away the best results and a net reduction of cellulite and the adipose tissues treated.

It has also been observed that the positive effects of reducing cellulite and adipose tissues associated with cavitation are maximised when the cavitation occurs at low ultrasonic frequencies.

Indeed, it is known that the cavitation phenomenon has intensity and efficacy inversely proportional to the frequency at which the same occurs.

However, said low frequencies must remain above 25 kHz so as not to cause unwelcome noise.

Furthermore, it has been observed that the thermal effects caused by ultrasounds do not produce significant results, from the point of view of reducing cellulite and adipose tissues.

Hence, the method according to the invention envisages that the ultrasonic frequencies described are appropriately propagated inside the human or animal body by means of a special device according to the invention, indicated overall by the number **1.**

Said device, which is described in detail hereinafter, mainly consists of a generating device **10,** capable of generating, or more simply emitting, an alternating electrical current, and one or more transducers **30,** electrically connected to the generating device 10 and suitable for transforming the alternating electrical current into mechanical vibrations of the frequencies indicated, giving rise to the aforementioned ultrasonic frequencies.

When activated, the transducers 30, upon coming into contact with an aqueous liquid, give rise to the aforementioned cavitation phenomena. Said transducers 30 are then appropriately placed in contact with the patient undergoing treatment **2;** in particular, they are placed in contact with the epidermis **3** of said patient undergoing treatment 2 at the areas of the epidermis showing said adipose accumulations or cellulite **4.**

Said transducers 30 then produce the aforementioned cavitation phenomenon, at the frequencies indicated, which propagate inside the adipose accumulations or cellulite 4. Said adipose accumulations or cellulite 4 are then destroyed by the implosion of the cavitation bubbles. The destroyed cells are then subsequently expelled by means of the normal body metabolism; in particular, they are expelled in the form of liquid waste.

However, the method described has encountered a significant drawback.

Indeed, it has been observed that the thermal effects caused by ultrasounds at said frequencies reach values that are poorly tolerated by the patients undergoing treatment.

In particular, it has been observed that the transducer device 30 reaches temperatures in excess of 50°C and close to 80°C.

According to the applicant's studies and tests, it has been determined that the temperature increase was mainly due to the presence of stationary waves, having fixed nodes and antinodes, in close proximity to which the thermal effects reach very high values.

Hence, with the aim of eliminating said stationary waves, the new method envisages the application of ultrasounds at frequencies that vary over time.

In particular, optimal results have been achieved with ultrasounds of a single frequency value, and wherein said frequency value is variable over time.

This frequency value, despite varying over time, in any case remains within the limits indicated above, particularly, it varies within a frequency band of between 2 kHz and 12 kHz, more particularly within an interval of around 6 kHz.

Hence, the frequency value preferably varies between 36 kHz and 42 kHz, more preferably between 38 kHz - 40 kHz.

Furthermore, this frequency varies appropriately according to regular cycles, particularly, the frequency value may vary linearly, or sinusoidally, or even otherwise over time. Appropriately, said cycles have duration periods preferably comprised of between 5.0 and 0.01 seconds, more appropriately around 0.3 - 0.2 seconds.

Despite varying in an approximately continuous manner, the frequency varies precisely by means of frequency micro-steps comprised of between 0.1 kHz and 0.01 kHz, appropriately around 0.05 kHz. Appropriately, said frequency micro-steps have time durations comprised of between 0.1 and 10 thousandths of a second.

The device 1, mainly consists of a generating device 10, capable of generating alternating electrical current and one or more transducers 30, electrically connected to the generating device 10 and capable of transforming the alternating electrical current into mechanical vibrations, giving rise to said ultrasounds. Said device is appropriately connected to the electricity supply by means of special connectors **11,** essentially consisting of an electrical cable and a plug.

The generating device 10 is regulated by special means of control **12,** appropriately operated manually and consisting for instance, of a keypad **13,** including a main power switch **13a** which makes or breaks the connection with the electricity supply, and a viewing screen **14** for the selected parameters.

Furthermore, the means of control preferably include a push-button switch **15** appropriately pedal-operated and connected by means of a special connection to the device 10. Said push-button switch is capable of actuating the transducer 30.

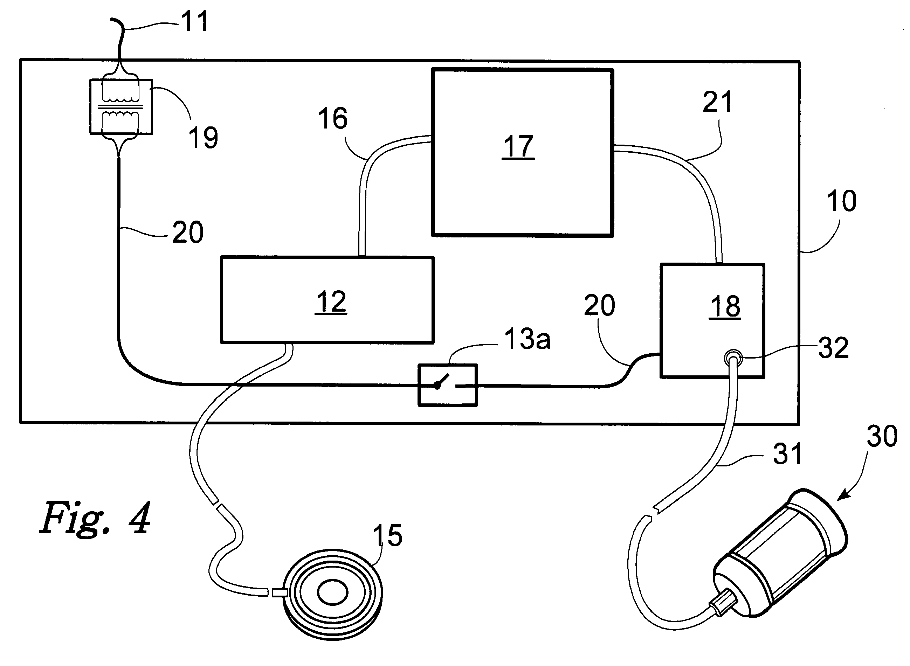

The means of control 12 are connected, by means of suitable first means of connection **16,** to a control and processing unit **17** (Fig. 4).

The control and processing unit 17 has the purpose of directing and controlling the correct operation of a power circuit **18,** adapted to amplifying the signals originating from the monitoring unit and supplying the transducers 30 with the necessary alternating electrical current. Said units 17 and circuit 18 are connected by means of second means of connection **21.**

Furthermore, the device 10 appropriately comprises an isolation transformer **19** directly connected to the electricity supply, and adapted to isolating the same from the power circuit 18 and/or the control and processing unit 17, in such a way that the vibrations and spikes of the electricity supply have no effect on said circuit 18 and said unit 17.

More particularly, the control and processing unit 17 consists of a programmable frequency generator, appropriately consisting of a "programmable logic device (PLD)".

The control and processing unit 17 further consists of an internal non-volatile memory, appropriately of the flash-memory type, which is programmed through the means of control 12.

The electricity supply for the control and processing unit 17 is preferably provided by the power circuit 18 and preferably has a voltage of approx. 5V.

Instead, the power circuit 18 is preferably supplied by an isolation transformer 19, by means of third means of connection **20,** and it additionally preferably comprises a resonant half-bridge inverter adapted to generating sinusoidal waves.

Thus, said inverter provides the electrical signal necessary for activating the transducers 30. Said electrical signal is propagated by means of detachable electrical connectors **31** which can convey it to the transducer 30.

The detachable electrical connectors 31, essentially consisting of an electrical cable and a mobile connection **32** such as an electrical plug and socket, has sufficient length or capacity to allow convenient use of the transducer 30.

Thus, the transducers 30 transform the alternating electrical signal originating from the power circuit 18 into mechanical vibrations of the same frequencies.

Particularly, it is well known that such transducers 30 possess resonance frequencies. Whenever they are stimulated by alternating electrical current with frequencies close to the resonant frequency, they incite particularly high amplitude mechanical vibrations, which allow the establishment of the cavitation phenomenon.

The present device 1 is also based on the physical principle described.

In particular, the transducers 30 are realized in such a way as to obtain resonant frequencies corresponding to the 25 kHz to 50 kHz frequency interval, more particularly corresponding to the 36 kHz - 42 kHz frequency interval. Indeed, whenever cavitation is achieved within such vibration intervals, as described previously, the positive cavitation-linked effects on reducing adipose tissues and cellulite are maximised.

The resonant frequencies of the transducers 30 are defined by numerous variables, such as in particular, the dimensions of the various components of which they are made, as specified below.

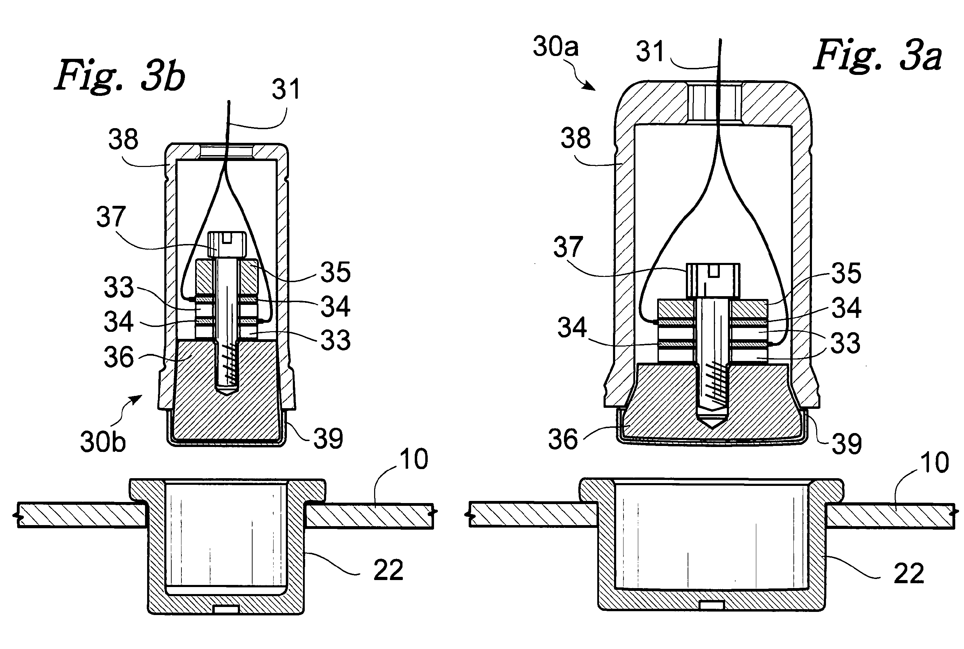

These are shown in cross-section in Figures 3a and 3b. Additionally, two transducers 30 of different sizes are envisaged; one larger **30a** intended to be used on the lower limbs and abdomen, and one smaller **30b** intended for use on then upper limbs, having the dimensions indicated below.

Each of them consists of a plurality of piezoelectric units **33.**

Said piezoelectric units 33 are made from piezoelectric ceramic material, preferably realized by a lead-titanium intermetallic ceramic commonly known as "lead-titanate". These are preferably disk-shaped, and are furthermore preferably arranged in stacks, and electrically connected to detachable electrical connectors 31 by means of appropriate conductors **34.**

The conductors 34, which are electrically connected to detachable electrical connectors 31, are themselves appropriately disk-shaped and made from an alloy of copper or other conducting metals, and interposed between said stacked piezoelectric units 33.

In addition, the transducer 30 preferably consists of a block body **35,** itself also disk-shaped, and an essentially truncated cone shaped diffuser base **36,** both of which are made from metallic materials, preferably steel.

The block body 35 and the diffuser base 36 are preferably stacked at both ends of the piezoelectric units 33 and the conductors 34, and enclose the same by means of specific means of fixing **37,** appropriately consisting of an Allen screw engaging with said diffuser base 36.

The diffuser base 36 has the purpose of diffusing the mechanical vibrations created by the piezoelectric units 33, while the block body 35 and the means of fixing 37, have the purpose of allowing the correct channelling of said mechanical vibrations towards the diffuser base.

With the aim of allowing the fixing of the block body 35, the piezoelectric units 33, the conductors 34 and the diffuser base 36, the components themselves have a central passing hole, through which the means of fixing 37 pass.

In particular, the means of fixing 37 engage with said block body 35, appropriately by means of the head of the means 37 having dimensions greater than the dimensions of the through hole formed in said block body, and in said diffuser base 36 having a blind, threaded, through hole. Hence, the means of fixing 37 sandwich the various components making up the transducer 30.

The dimensions of the various components making up the transducers 30 are specified below.

In particular, the larger transducer 30a has piezoelectric units 33 having external diameters comprised of between 29 mm and 23 mm, appropriately around 26 mm, and height comprised of between 4 mm and 6 mm, appropriately around 5 mm, conductors of the same diameter with height one millimetre shorter; a diffuser base 36 having a broader base, facing outwards, with dimensions comprised of between 39 mm and 45 mm more preferably around 42.3 mm, a narrower base, facing onto the unit 33, with dimensions comprised of between 33 mm and 39 mm more preferably around 36 mm and with height comprised of between 15 mm and 20 mm and preferably around 17.5 mm; a block body with a diameter similar to the diameter of the unit 33 and height comprised of between 4 mm and 10 mm; and finally means of fixing 37 consisting of an Allen screw having a length comprised of between 18 mm and 28 mm, appropriately 23 mm, and a diameter comprised of between 6 mm and 10 mm, appropriately around 8 mm, the diameter of said screw appropriately defining the same diameter of the through holes present in the centre of the unit 33, of the conductors 34, the block body 35 and the diameter of the blind, threaded through hole in the diffuser base 36.

On the other hand the smaller transducer 30b has piezoelectric units 33 having external diameters comprised of between 13 mm and 17 mm, appropriately around 15 mm, an a height comprised of between 6 mm and 10 mm, appropriately around 8 mm, conductors of the same diameter with height one millimetre shorter; a diffuser base 36 having a wider base facing outwards, of dimensions comprised of between 21 mm and 27 mm more preferably around 24 mm, a narrower base facing onto the unit 33, of dimensions comprised of between 23 mm and 30 mm more preferably around 26.3 mm and a height comprised of between 20 mm and 26 mm and preferably around 23 mm; a block body with diameter similar to the diameter of the units 33 and height comprised of between 4 mm and 10 mm; and finally means of fixing 37 consisting of an Allen screw having a length comprised of between 23 mm and 29 mm, appropriately 26 mm, and a diameter comprised of between 4 mm and 8 mm, appropriately around 6.25 mm, the diameter of said screw appropriately defining an identical diameter of through holes present at the centre of the units 33, the conductors 34, the block body 35 and the diameter of the blind threaded through hole in the diffuser base 36.

Finally, the transducers 30 are appropriately inserted inside a capsule consisting of a cushioned handle **38**, preferably realized by polymer material, appropriately polyvinylchloride (PVC), and adapted to quenching the vibrations originating from the unit 33, and a diffuser **39**, preferably made of metal, more preferably 303 steel, and adapted to facilitating contact with the epidermis 3a of the patient undergoing treatment 2.

The diffuser 39 of the larger transducer 30a has an outer diameter comprised of between 40 mm and 48 mm, preferably around 44 mm; while the diffuser 39 of the smaller transducer 30b has an outer diameter comprised of between 25 mm and 31 mm, preferably around 28 mm.

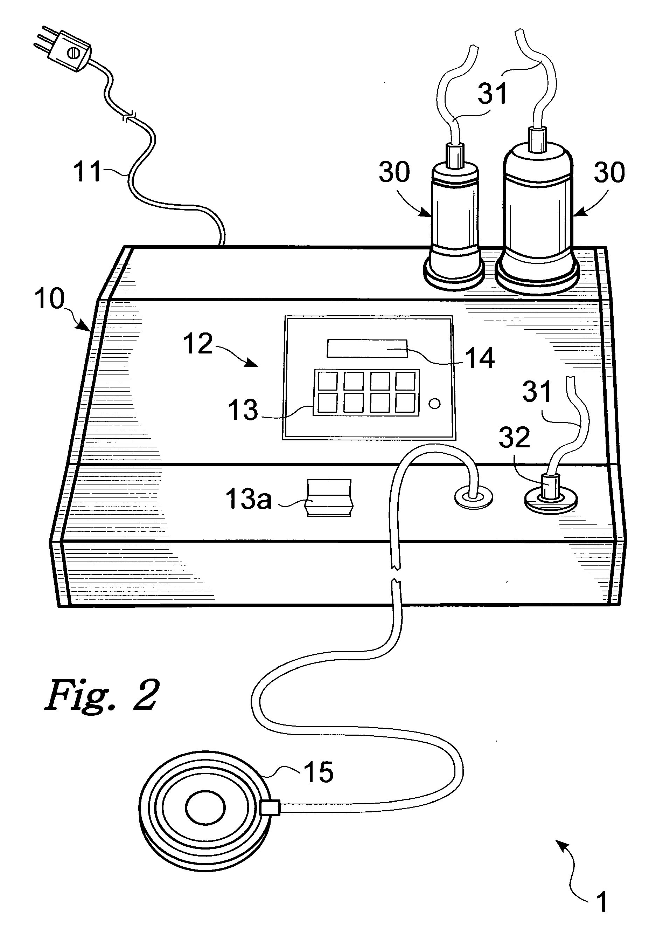

Finally, the device 10 has housings **22** into which the transducers 30a and 30b, may be arranged, as shown in Fig. 2.

The operation of the device **1**, described structurally above, is as follows.

The apparatus 1 is connected to the electricity supply by means of the connectors 11 and switched on by means of the switch 13a.

Furthermore, said apparatus 1 is adjusted using the means of control 12, particularly by use of the keypad 13. In particular, the following are adjusted: the frequency variation cycle period, the frequency variation modes, the application duration, the vibration frequencies and more besides.

The selected parameters are appropriately viewed on the viewing screen 14. The inserted settings are stored in the non-volatile memory of the control and processing unit 17 which processes the selected frequency signals at the selected times.

The operator administering the treatment to the patient 2 holds the transducer 30, connected to the apparatus 10, by means of the cushioned handle 38 and, by applying pressure to the push-button switch 15, activates the power circuit 18, which amplifies the signal originating from the control and processing unit 17 and transmits it to the transducer 30. The transducer 30 then begins to emit ultrasounds with the previously described variable frequencies.

In order to perform treatment, the diffuser 39 of the transducer 30 is placed in contact with the epidermis 3 of the patient undergoing treatment 2, in the areas affected by adipose tissues or cellulite.

An aqueous contact gel, in which the ultrasonic frequencies propagate, is spread on the epidermis of the patient in advance.

The transducer 30 transmits the ultrasounds inside the adipose tissues or cellulite, causing cavitation within the same, and hence their destruction.

The destroyed adipose cells are subsequently expelled by means of normal body metabolism.

The invention achieves important advantages.

Indeed, it has allowed significant aesthetic improvements with means that are neither invasive nor tiring for the patient undergoing treatment.

Furthermore, administration times are greatly reduced; indeed weekly sessions lasting approximately one hour are sufficient to obtain significant results.

## Claims

1. A method for the aesthetic treatment of cellulite and the adipose tissues present in areas of human or animal bodies, consisting of the application of ultrasounds to said body parts, and **characterised in that** said ultrasounds are of a frequency comprised of between 25 kHz and 50 kHz and capable of generating cavitation within aqueous liquids.

2. The method according to claim 1, wherein said ultrasounds are of a frequency comprised of between 36 kHz and 42 kHz.

3. The method according to claim 1, wherein said ultrasounds have a single frequency value, and where said frequency value varies continuously over time within a frequency band comprised of between 2 kHz and 12 kHz.

4. The method according to claim 3, wherein said frequency value of said ultrasounds varies in accordance with regular cycles.

5. The method according to claim 4, and wherein said cycles have a duration comprised of between 2.0 and 0.01 seconds.

6. The method according to claim 3, wherein said frequency value varies linearly over time.

7. A device for.the treatment of cellulite and adipose tissues present in areas of human or animal bodies comprising: an alternating electrical current generating device (10), at least one transducer (30), electrically connected to said generating device (10) and suitable for transforming said alternating electrical current into mechanical vibrations generating ultrasounds, means of control (12) of said generating device, **characterised in that** said transducer is adapted to generating ultrasounds of a frequency comprised of between 25 kHz and 50 kHz and suitable for generating cavitation within aqueous liquids.

8. The device according to claim 7, wherein said generating device (10) is adapted to generating alternating electrical current with a single frequency value, and wherein said frequency value varies continuously over time within a band comprised of between 2 kHz and 12 kHz.

9. The device according to claim 7, wherein said transducer (30) has a resonant frequency comprised of between 25 kHz and 50 kHz.

10. The device according to claim 7, having two of said transducers (30) a larger transducer (30a), with dimensions suitable for treating the lower limbs and abdomen of said human body, and a smaller transducer (30b), with dimensions suitable for treating the upper limbs of said human body.
